# FASCICULE DE BREVET EUROPEEN

(11) **EP 1 731 098 B1**
(45) Date de publication et mention de la délivrance du brevet: **21.01.2009**
(21) Numéro de dépôt: 06115009.0
(22) Date de dépôt: 06.06.2006
(51) Int. Cl.: A61B 5/11

(54) **Procédé de détection de chute d'une personne**
Verfahren zur Erfassung des Sturzes einer Person
Method for detecting the fall of a person

(30) Priorité: 07.06.2005 FR 0551521
(43) Date de publication de la demande: 13.12.2006
(73) Titulaire: COMMISSARIAT A L'ENERGIE ATOMIQUE, 75015 Paris (FR)
(72) Inventeur: Bonnet, Stéphane, 38170, Seyssinet (FR); Guillemaud, Régis, 38700, La Tronche (FR)
(74) Mandataire: Poulin, Gérard

(56) Documents cités:
- EP-A- 1 493 385
- FR-A- 2 829 862
- US-A1- 2002 008 630
- US-A1- 2002 103 610
- B KEMP, A JANSSEN, B VAN DER KAMP,: ""Body position can be monitored using miniature accelerometers and earth magnetic field sensors"" ELECTROENCEPHA. CLINICAL NEUROPHYSIOLOGY, vol. 109, décembre 1998 (1998-12), pages 484-488, XP002367000

## Description

L'invention a trait à un procédé et un système de détection de chute d'une personne. Elle trouvera emploi notamment dans des institutions médicalisées prenant en charge des personnes âgées.

Il existe déjà un art antérieur abondant de systèmes et de procédés pour suivre l'activité d'une personne et éventuellement détecter un état critique demandant une intervention. On utilise généralement un capteur ou plusieurs capteurs qu'on place sur la personne et qui délivrent continuellement des signaux représentatifs de son activité physique. Ces signaux comprennent typiquement des mesures d'accélération suivant un ou plusieurs axes : une chute apparaît alors comme une variation brusque comprenant généralement une série d'oscillations de courte durée sur au moins un des signaux d'accélération. Un document représentatif est le brevet US-B2-6 703 939.

La demande de brevet EP 1 493 385 décrit un procédé de détection de chute d'une personne, comprenant les étapes suivantes:
- placer sur la personne un capteur comprenant un accéléromètre et un magnétomètre;
- suivre continuellement les signaux d'accélération et de champs magnétique donnés par le capteur;
- indiquer une chute quand l'un des critères a été constaté: a) une période d'instabilité du signal de l'accélération, ou b) une variation du signal de champs magnétique ambiant.

Un écueil qu'on rencontre toujours avec des systèmes de ce genre est qu'il est difficile de déterminer la nature des événements accomplis ou subis par le porteur du capteur d'après les signaux qu'il émet. Si une chute est caractérisée par une accélération en direction verticale, des actions de se baisser, de s'asseoir, etc. le sont aussi. En se couchant, le porteur reproduit même de façon naturelle un mouvement ressemblant à une chute. On a imaginé certains critères de discrimination entre une chute accidentelle et de tels événements normaux, d'après des seuils des oscillations du signal par exemple, mais ils ne sont pas suffisants. Des perfectionnements sont donc attendus par les spécialistes. L'invention en représente un. Dans le procédé de détection de chute qu'elle constitue, la mesure du signal d'accélération est complétée par la mesure de champ magnétique ambiant, selon la revendication 1. Une telle combinaison donne une indication plus fiable de la chute.

L'invention implique l'emploi d'au moins un magnétomètre dans le capteur placé sur la personne, en supplément de l'accéléromètre. Le magnétomètre mesure, le champ magnétique ambiant et donne donc une mesure complètement différente de celle de l'accéléromètre, de l'activité de la personne porteuse. Les mesures délivrées par le magnétomètre ne sont pas sensibles aux accélérations du capteur mais seulement aux changements d'orientation. Celles qui sont délivrées par le magnétomètre dépendent de l'orientation du capteur dans l'espace : par exemple, lorsqu'il n'y a pas de perturbation magnétique telle que la présence d'un objet ferromagnétique à proximité de la personne, on mesure sur chacun des axes sensibles du capteur la projection du champ magnétique terrestre. Il a notamment été constaté que, pendant la période d'instabilité du signal d'accélération, caractéristique d'une chute comme de certaines activités normales de la personne, la mesure de certaines composantes du signal de champ magnétique ambiant révélait une transition progressive entre deux niveaux différents de mesure du champ magnétique, stable avant et après la période d'instabilité, quand une chute arrivait vraiment, et la mesure du signal d'accélération et la différence de mesures du champ magnétique avant l'instabilité et après l'instabilité donnaient à elles seules de bonnes chances d'indication exacte de la chute en éliminant un bon nombre d'événements normaux qui étaient confondus avec elle.

L'invention peut être appliquée de bien des façons. D'autres critères peuvent tout d'abord être ajoutés pour compléter l'évaluation de l'occurrence d'une chute et donner une indication encore plus fiable.

Ces critères peuvent généralement, dans des signaux mesurés et suivis continuellement, être validés si les variations de ces signaux dépassent un seuil pendant une durée définie, en terme d'intensité ou d'énergie du signal par exemple.

Des signaux qui seront favorablement mesurés sont la composante verticale d'accélération et la composante verticale du champ magnétique ambiant, mesurées dans un repère lié à la personne, de la tête aux pieds, et non mesurées dans un repère terrestre. La chute d'une station debout s'accompagne toujours d'une telle accélération verticale, et la mesure de la composante verticale du champ magnétique ambiant varie d'une valeur stable à la station debout à une valeur généralement différente après une chute, la composante verticale liée à la personne étant alors incluse dans un plan terrestre horizontal.

Il est compris que, pour que les critères précédents et d'autres soient tenus pour fiables, la période d'instabilité du signal d'accélération doit être suivie d'une période stable de durée suffisante des signaux, qui peut impliquer une perte de connaissance ; l'événement lié à l'instabilité est sinon tenu pour dépourvu de gravité et négligé par le procédé, quelle que soit sa nature.

D'autres critères de détection de la chute peuvent comprendre une détection de position couchée quand la stabilité du signal d'accélération a été obtenue, par exemple en mesurant une accélération due à la gravité à une valeur sensiblement nulle dans la direction verticale liée à la personne.

Les critères peuvent aussi comprendre une reconnaissance de certains événements normaux susceptibles d'être confondus avec une chute. Un tel événement courant sera la mise au lit de la personne. Le critère de reconnaissance peut comprendre, pour cet évènement ou d'autres, une mesure d'azimut de la personne, c'est-à-dire de son orientation dans un plan horizontal. Dans des circonstances usuelles d'emploi de l'invention, la personne porteuse ne dispose que d'un tout petit nombre d'endroits où se coucher, qu'il est possible de repérer facilement par des mesures préliminaires. Une chute se produira à un azimut quelconque, contrairement à un coucher normal où l'orientation de la personne sera déterminée à un azimut bien connu.

La discrimination par reconnaissance d'événements normaux pourra se faire non seulement par la mesure d'un état final, mais par l'examen de toute une portion de signal, puisque les événements tels que la mise au lit s'effectuent généralement par des gestes invariables qui donnent un déroulement électronique caractérisé des signaux, c'est-à-dire une signature électronique.

D'une façon générale, la chute pourra être indiquée par une combinaison numérique et pondérée des critères retenus. Si un seuil numérique est franchi par cette combinaison, la chute sera indiquée et l'alerte pourra être donnée.

L'invention sera maintenant décrite en liaison aux figures :
- la figure 1 illustre l'utilisation du capteur,
- la figure 2 est une vue d'ensemble du système,
- la figure 3, comprenant des diagrammes 3A, 3B et 3C, illustre une réalisation fondamentale de la détection,
- la figure 4, comprenant des diagrammes 4A, 4B et 4C, illustre une détection particulière dans le procédé,
- et la figure 5 est un diagramme récapitulatif du procédé.

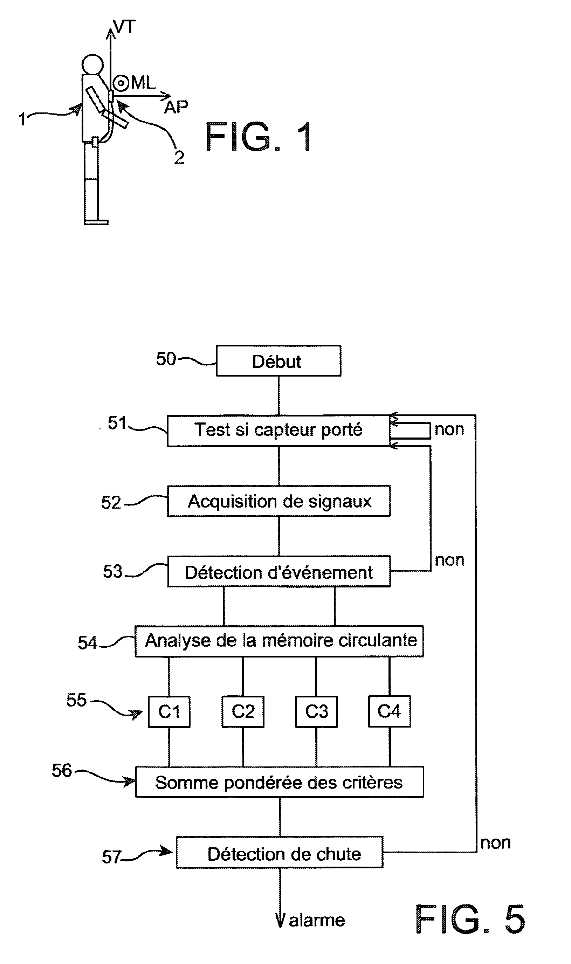

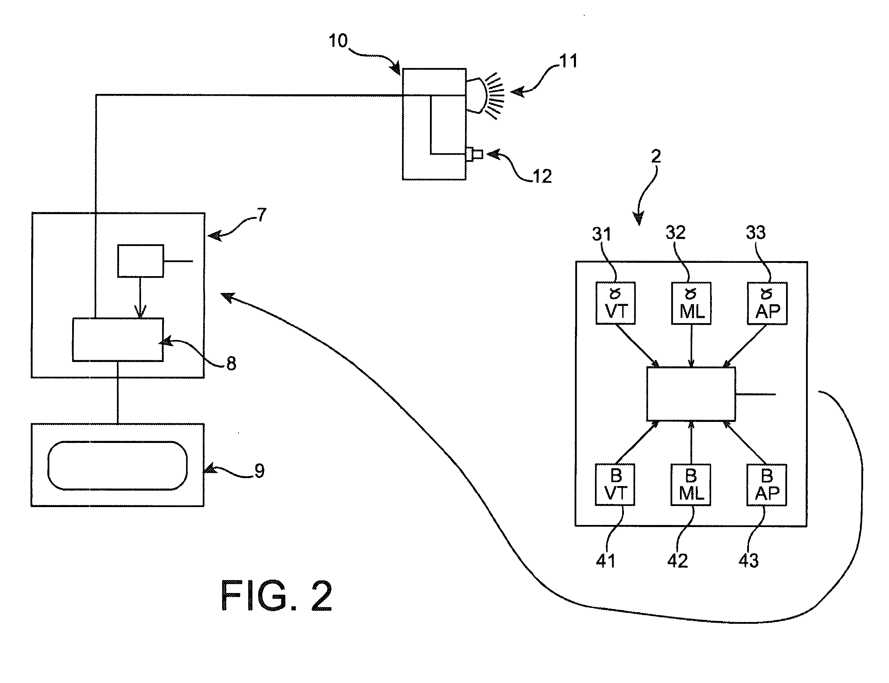

La figure 1 représente une personne 1 à surveiller d'après le procédé et sur laquelle on peut définir un repère formé d'un axe vertical VT dirigé des pieds à la tête, un axe antéro-postérieur AP dirigé vers l'avant et un axe médio-latéral ML dirigé vers le côté. L'origine des axes est supposée sur le tronc de la personne 1, par exemple la poitrine ou une hanche, où est placé un capteur 2 dont une réalisation est illustrée plus en détail à la figure 2. Elle comprend trois accéléromètres 31, 32 et 33 mesurant les accélérations qu'ils subissent, gravité incluse, dans les trois axes orthogonaux du repère et trois magnétomètres 41, 42 et 43 qui mesurent les composantes du champ magnétique ambiant, essentiellement le champ magnétique naturel terrestre, dans les trois axes orthogonaux du repère. Il importe de placer le capteur 2 suivant une bonne orientation sur la personne 1, pour que les axes de mesures des accéléromètres 31, 32, 33 et des magnétomètres 41, 42 et 43 coïncident bien avec les directions du repère de la personne 1. En variante, le capteur 2 pourrait être placé à une orientation quelconque mais mesurée par un procédé de calibration (en plaçant la personne 1 à une position déterminée par rapport à un repère terrestre et en exploitant les mesures de capteur 2 à cette position), et les mesures des capteurs seraient converties pour être données dans les trois axes du repère. Les mesures, converties ou non, des accéléromètres 31, 32, 33 et des magnétomètres 41, 42, 43 sont transmises via un émetteur-récepteur à une station d'examen 7 qui comprend en particulier un processeur 8 de détection de chute dont le fonctionnement sera décrit ensuite. Dans un premier mode de réalisation, quand une chute est détectée, cela est transmis à un dispositif afficheur 9 local pour donner l'alerte et à un dispositif d'alarme 10 pouvant comprendre des lampes 11, des bruiteurs, etc. et un bouton 12. Le dispositif d'alarme 10 est placé dans le lieu où vit la personne 1, qui a la faculté d'arrêter une alerte intempestive en appuyant sur le bouton 12. Dans un premier mode de réalisation, le dispositif d'alarme est relié à un centre de téléassistance médicale en vue de déclencher les secours à la personne 1. Dans un deuxième mode de réalisation plus avantageux, les moyens de traitement sont embarqués : le dispositif de commande de l'alarme est situé sur le corps du patient.

Une instabilité forte en amplitude, en durée ou en fréquence, mesurée sur les accéléromètres et en particulier sur l'accéléromètre vertical, est associée à un mouvement induisant un impact sur le capteur (chute, marche, saut, etc.). Une variation de la valeur de la projection du champ magnétique sur les axes du capteur est associée à un changement d'orientation de la personne, comme tel est le cas dans une chute, une mise au lit, ou l'action de se pencher.

L'occurrence des deux événements dans une plage de temps limitée, de l'ordre de grandeur de quelques secondes, par exemple 2 secondes, donne une présomption forte de chute.

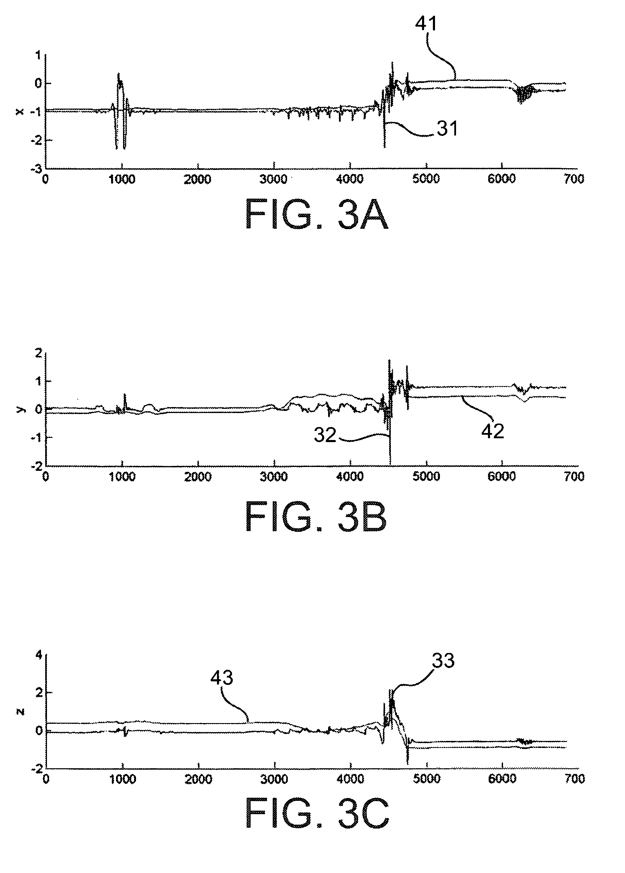

Les mesures des capteurs peuvent être celles qui sont données à la figure 3, où le premier diagramme 3A donne les mesures de l'accéléromètre 31 et du magnétomètre 41 dans l'axe vertical VT, le diagramme 3B donne les mesures de l'accéléromètre 32 et du magnétomètre 33 dans l'axe médio-latéral ML, et le diagramme 3C donne les mesures de l'accéléromètre 33 et du magnétomètre 43 dans l'axe antéro-postérieur AP. La gamme de mesures des capteurs peut être ±5g environ pour les accéléromètres, 50 microteslas environ pour les magnétomètres. Des détecteurs existent sur le marché et peuvent être utilisés sans empêchement particulier. Un exemple est l'accéléromètre triaxial LIS3L02AQ de ST Micro Electronics. Dans cet exemple, les mesures d'accélération donnent des périodes prépondérantes de stabilité coupées de périodes plus courtes d'instabilité où l'accélération donne des oscillations importantes et brèves. Ces périodes d'instabilité sont au nombre de deux principalement ici et correspondent à deux événements différents : le premier, enregistré autour du temps t=1000, est un bond de la personne 1 ; l'autre, enregistré entre les temps t=4000 et 5000 environ, est une chute. Ces deux événements sont assez peu distincts en ce qui concerne les mesures d'accélérations, surtout dans l'axe vertical VT qui est précisément celui qui permet de reconnaître le plus facilement une chute ; mais on observe que les mesures des magnétomètres 41, 42 et 43 sont presque insensibles au bond, alors qu'elles relèvent une variation importante à la chute, qui est particulièrement visible dans la composante verticale du diagramme 3A. Plus précisément, la mesure du magnétomètre 41 dans l'axe vertical VT présente deux paliers à des niveaux différents avant et après la chute, que relie une variation progressive et assez régulière aux instants d'instabilité du signal d'accélération. Les diagrammes 3B et 3C montrent qu'une conclusion équivalente de l'occurrence d'une chute peut généralement être obtenue de la même façon avec des magnétomètres 42 et 43 placés dans d'autres axes que le vertical VT ; une exception apparaît cependant quand la chute n'implique pas de variation de l'angle que fait l'axe vertical avec la direction du champ magnétique ambiant, ce qui peut inciter à mesurer le champ magnétique avec au moins deux magnétomètres placés dans des directions différentes pour plus de sûreté ; ici on remarque que la variation d'intensité de la mesure du magnétomètre 42 dans l'axe médio-latéral ML est peu importante, ce qui peut faire que la chute sera inaperçue si on ne considère que les mesures associées à cet axe.

On cherche à obtenir une bonne discrimination entre une chute accidentelle et d'autres événements qui lui ressemblent par les signaux émis. Un critère supplémentaire qu'il est intéressant de mettre en oeuvre pour cela est de vérifier que la personne 1 est étendue après la chute, ce qui sera vrai sauf à de rares exceptions.

La position étendue est vérifiée si l'accéléromètre 31 dans l'axe vertical VT donne une mesure à peu près nulle après l'événement supposé être une chute, ce qui indique qu'il est perpendiculaire à la direction de la gravité. Cette vérification est entreprise ici après le temps t=5000 environ.

On peut tenter de distinguer la chute d'une action volontaire de se coucher de la personne 1, et notamment d'une mise au lit. Ici aussi, les mesures magnétométriques sont utiles, puisque l'azimut de la personne 1, ou son orientation dans un plan horizontal terrestre, sera uniforme à quelques degrés près quand elle sera couchée normalement au lit. Une mesure des résultats des magnétomètres 41 et 42 disposés dans l'axe vertical VT et l'axe médio-latéral ML est entreprise à partir du même instant t = 5000 environ pour en déduire cet azimut de la personne 1. S'il est différent de celui de la position couchée normale, la chute devra être présumée.

Si la référence d'azimut final manque, un critère presque aussi intéressant peut être obtenu en comparant les variations d'azimut de la position initiale à la position finale.

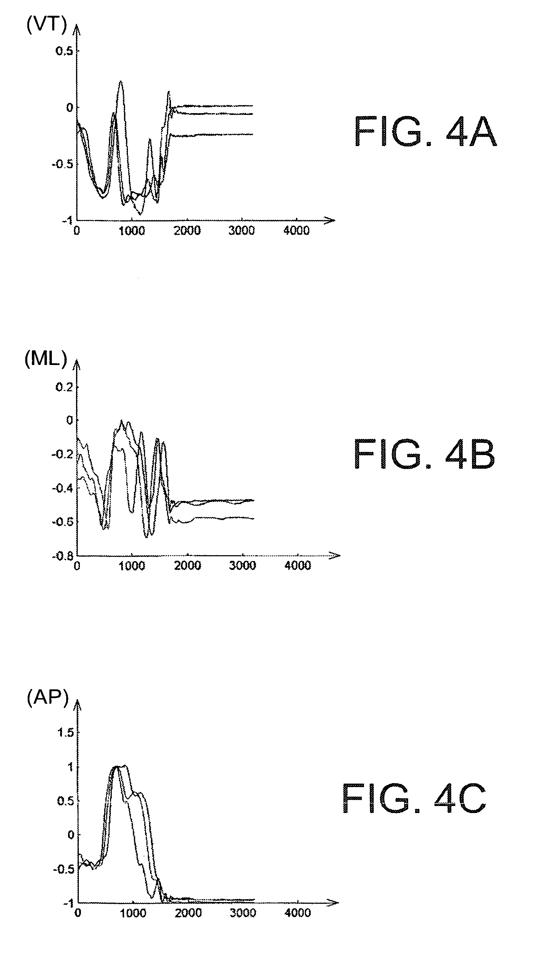

Il est encore possible d'utiliser le capteur 2 pour reconnaître cet événement normal de mise au lit. Les diagrammes 4A, 4B et 4C de la figure 4 illustrent respectivement les résultats des magnétomètres 41, 42 et 43 dans les axes vertical VT, médio-latéral ML, et antério-postérieur AP pour une série de mises au lit de la personne 1. On constate que les signaux enregistrés à des moments différents pour le même acte se ressemblent beaucoup et peuvent donner une signature électronique de cet acte, la personne 1 accomplissant à peu près toujours les mêmes gestes. On reconnaît une phase où elle s'assied entre les temps t = 0 et t = 700 environ (au diagramme 4A), tout en se tournant (au diagramme 4B), et en se penchant vers l'avant (à partir de t = 500 environ au diagramme 4C), avant de se tourner de nouveau jusqu'à t = 1700 environ (au diagramme 4B), et de se pencher en arrière (aux diagrammes 4A et 4C). La signature qu'on enregistre par des calibrations préliminaires peut concerner les deux parties, distinguées ci-dessus, de l'événement ou seulement la seconde, plus caractéristique. On comparera ensuite ces signatures aux portions intéressantes de signaux homologues, obtenus de la même façon avec les mêmes détecteurs et le même environnement, en recourant à des techniques classiques de corrélation de signaux.

La figure 5 illustre une réalisation du procédé. Après une initialisation à l'étape 50, un test 51 pour savoir si le capteur 2 est porté est fait périodiquement. Dans l'affirmative, les mesures du capteur 2 sont entreprises à l'étape 52. Les résultats sont maintenus dans une mémoire circulante du processeur 8 pendant cinq minutes environ. Si aucun événement n'est enregistré à l'étape suivante 53, on revient à l'étape 51, et le système travaille en boucle. Mais quand un événement est enregistré à l'étape 53, comme une variation brusque du signal d'un accéléromètre, une variation significative des signaux des magnétomètres ou une détection de posture allongée, une analyse 54 de la mémoire circulante est entreprise d'après les critères indiqués précédemment ou certains d'entre eux. Ces critères peuvent être, dans le cas présent, ramenés à quatre critères numériques C1, C2, C3 et C4 calculés dans une étape 55 et prenant une valeur de 0 ou 1 selon qu'on estime qu'ils sont absents ou présents ; des valeurs intermédiaires, correspondant à une analyse en logique floue, pourraient être proposées dans des cas douteux.

Le nombre de critères utilisés dépend de la fiabilité de résultat désirée ainsi que de la qualité et de la puissance des moyens de traitement. Plus on aura de critères et plus la fiabilité sera bonne.

Le critère C1 correspond à la détection que la personne s'est couchée par l'examen du signal d'accélération verticale. Il est validé à 1 s'il est vérifié pendant une durée de temps définie. Si une posture allongée a déjà été vérifiée, ce critère est validé sans condition d'ancienneté.

Le critère C2 correspond au passage d'une période d'activité forte de la personne 1, se traduisant par des oscillations ou des variations importantes des signaux, à un état d'activité faible. Si l'activité faible se maintient pendant une durée définie, ce critère est validé à 1. Le signal d'accélération dans l'axe vertical VT est utilisé. D'autres signaux peuvent être utilisés.

Le critère C3 correspond à la comparaison de l'azimut de la personne après l'événement et de l'azimut de la position couchée normale. Si ces azimuts sont différents au-delà d'une certaine tolérance, le critère est validé à 1. On a vu que les magnétomètres 41 et 42 étaient utilisés.

Le critère C4 correspond enfin à la comparaison du mouvement de l'événement aux signatures déjà enregistrées de certains mouvements normaux typiques, suivant les explications données à la figure 4. Si ces mouvements sont différents au-delà d'un seuil, le critère est validé à 1.

L'étape suivante 56 est une somme pondérée des critères C1 à C4, selon la formule
S= w1C1+w2C2+w2C2+w3C3=w3C4+w3C4, où la somme des coefficients de pondération w1 à w4 est égale à 1. Si la somme S est supérieure à un seuil, pouvant être 0,5 mais qu'on peut choisir selon la sensibilité recherchée et l'étroitesse de la surveillance de la personne 1, l'alarme est déclenchée après l'étape de détection de chute 57. Le système revient à l'étape 51 et continue les mesures quel que soit le diagnostic.

## Revendications

1. Procédé de détection de chute d'une personne, comprenant les étapes suivantes :
- placer sur la personne (1) un capteur (2) comprenant au moins un accéléromètre et au moins un magnétomètre,
- suivre continuellement un signal d'accélération et un signal de champ magnétique ambiant donnés par le capteur, et analyser leurs évolutions,
- indiquer une chute quand une pluralité de critères a été constatée, lesdits critères comprenant :
a) une période d'instabilité du signal d'accélération, suivie d'une stabilité du signal d'accélération ;
b) une variation progressive du signal de champ magnétique ambiant pendant la période d'instabilité du signal d'accélération, entre deux niveaux différents du signal de champ magnétique ambiant mesurés avant et après la période d'instabilité du signal d'accélération.

2. Procédé de détection de chute d'une personne selon la revendication 1, **caractérisé en ce que** le signal d'accélération comprend au moins une composante verticale d'accélération, dans une direction verticale (VT) liée à la personne.

3. Procédé de détection de chute d'une personne selon la revendication 1 ou 2, **caractérisé en ce que** le signal de champ magnétique ambiant comprend une composante verticale de champ magnétique ambiante dans une direction verticale (VT) liée à la personne.

4. Procédé de détection de chute d'une personne selon l'une quelconque des revendication 1 à 3, **caractérisé en ce que** les critères comprennent aussi :
c) une détection de position couchée à la stabilité du signal d'accélération, par une mesure de valeur sensiblement nulle dans une direction verticale (VT) liée à la personne.

5. Procédé de détection de chute d'une personne selon l'une quelconque des revendications 1 à 4, **caractérisé en ce que** les critères comprennent aussi :
d) une reconnaissance d'événements normaux susceptibles d'être confondus avec une chute.

6. Procédé de détection de chute d'une personne selon la revendication 5, **caractérisé en ce que** la reconnaissance comprend une mesure d'azimut de la personne.

7. Procédé de détection de chute d'une personne selon l'une quelconque des revendications 5 ou 6, **caractérisé en ce que** la reconnaissance comprend une comparaison d'au moins un des signaux à un signal homologue, enregistré auparavant et correspondant à un des événements normaux, pour la période d'instabilité du signal d'accélération.

8. Procédé de détection de chute d'une personne selon l'une quelconque des revendications 4 à 6, **caractérisé en ce que** les événements normaux comprennent une mise au lit.

9. Procédé de détection de chute selon l'une quelconque des revendications précédentes, **caractérisé en ce que** la chute est indiquée d'après le franchissement d'un seuil (S) par une combinaison numérique et pondérée des critères.

10. Procédé de détection de chute selon l'une quelconque des revendications précédentes, **caractérisé en ce qu'**il comprend une conversion de mesures d'après une orientation du capteur (2), pour exprimer les mesures dans un repère d'axes lié à la personne et comprenant un axe vertical, un axe antéro-postérieur et un axe médio-latéral, et comprenant une calibration du capteur pour déterminer ladite orientation.

## Claims

1. Procedure for detecting the fall of a person, comprising of the following stages:
- placing on the person (1) a sensor (2) comprising at least one accelerometer and at least one magnetometer,
- continuously monitoring an acceleration signal and an ambient magnetic field signal provided by the sensor, and analysing their changes,
- indicating a fall when a plurality of criteria have been met, such criteria including:
a) a period of instability in the acceleration signal, followed by stability in the acceleration signal;
b) a gradual change in the ambient magnetic field signal during the instability period of the acceleration signal, between two different levels of the ambient magnetic field signal measured before and after the instability period of the acceleration signal.

2. Procedure for detecting the fall of a person according to claim 1 or 2, **characterized by** the acceleration signal consisting of at least one vertical acceleration component along a vertical direction (VT) associated with the person.

3. Procedure for detecting the fall of a person according to claim 1, **characterized by** the ambient magnetic field signal including a vertical component of the field along a vertical direction (VT) associated with the person.

4. Procedure for detecting the fall of a person according to any one of claims 1 to 3, **characterized by** the fact that the criteria also include:
c) a detection of recumbent position in the stability of the acceleration signal by an effectively zero measurement along the vertical axis (VT) associated with the person.

5. Procedure for detecting the fall of a person according to any one of claims 1 to 4, **characterized by** the fact that the criteria also include:
d) a recognition of normal events likely to be confused with a fall.

6. Procedure for detecting the fall of a person according to claim 5, **characterized by** the fact that such recognition includes a measurement of the person's azimuth.

7. Procedure for detecting the fall of a person according to any one of claims 5 or 6, **characterized by** the fact that such recognition includes a comparison between at least one of the signals with an equivalent signal recorded previously and corresponding to one of the normal events, for the instability period of the acceleration signal.

8. Procedure for detecting the fall of a person according to any one of claims 4 to 6, **characterized by** the fact normal events include going to bed.

9. Procedure for detecting a fall according to any one of the proceeding claims, **characterized by** the fact that the fall is indicated after a threshold (S) is crossed by a weighted numerical combination of criteria.

10. Procedure for detecting a fall according any one of the proceeding claims, **characterized by** the fact that readings are converted according to the orientation of sensor (2) to express readings in a frame of axes associated with the person and consisting of a vertical axis, an antero-posterior axis and a medio-lateral axis, and including a calibration of the sensor to determine the aforementioned orientation.

## Patentansprüche

1. Verfahren zur Erfassung eines Sturzes einer Person, die folgenden Verfahrensschritte umfassend:
- Anbringen eines mindestens einen Beschleunigungsmesser und ein Magnetometer umfassenden Sensors (2) an der Person (1),
- ständiges Verfolgen eines Beschleunigungssignals und eines Umgebungsmagnetfeld-Signals, und Analysieren ihrer Entwicklungen,
- Melden eines Sturzes, wenn mehrere Kriterien festgestellt worden sind, wobei diese Kriterien umfassen:
a) eine Instabilitätsperiode des Beschleunigungssignals, gefolgt von einer Stabilität des Beschleunigungssignals;
b) eine progressive Veränderung des Umgebungsmagnetfeld-Signals während der Instabilitätsperiode des Beschleunigungssignals zwischen zwei verschiedenen Signalpegeln des Umgebungsmagnetfeld-Signals, gemessen vor und nach der Instabilitätsperiode des Beschleunigungssignals.

2. Verfahren zur Erfassung des Sturzes einer Person nach Anspruch 1, **dadurch gekennzeichnet, dass** das Beschleunigungssignal wenigstens eine vertikale Beschleunigungskomponente in einer an die Person gebundenen Vertikalrichtung (VT) umfasst.

3. Verfahren zur Erfassung des Sturzes einer Person nach Anspruch 1 oder 2, **dadurch gekennzeichnet, dass** das Umgebungsmagnetfeld-Signal eine vertikale Umgebungsmagnetfeld-Komponente in einer an die Person gebundenen Vertikalrichtung (VT) umfasst.

4. Verfahren zur Erfassung des Sturzes einer Person nach einem der Ansprüche 1 bis 3, **dadurch gekennzeichnet, dass** die Kriterien auch umfassen:
c) eine Liegepositionserfassung bei Stabilität des Beschleunigungssignals durch eine Quasi-Nullwert-Messung in einer an die Person gebundenen Vertikalrichtung (VT).

5. Verfahren zur Erfassung des Sturzes einer Person nach einem der Ansprüche 1 bis 4, **dadurch gekennzeichnet, dass** die Kriterien auch umfassen:
d) eine Erkennung von Normalereignissen, die mit einem Sturz verwechselt werden könnten.

6. Verfahren zur Erfassung des Sturzes einer Person nach Anspruch 5, **dadurch gekennzeichnet, dass** die Erkennung eine Messung des Azimuts bzw. eine Azimutmessung der Person umfasst.

7. Verfahren zur Erfassung des Sturzes einer Person nach einem der Ansprüche 5 oder 6, **dadurch gekennzeichnet, dass** die Erkennung für die Instabilitätsperiode des Beschleunigungssignals einen Vergleich von wenigstens einem der Signale mit einem vorher aufgezeichneten und einem der Normalereignisse entsprechenden homologen Signal umfasst.

8. Verfahren zur Erfassung des Sturzes einer Person nach einem der Ansprüche 4 bis 6, **dadurch gekennzeichnet, dass** die Normalereignisse einem Zubettgehen entsprechen.

9. Verfahren zur Erfassung des Sturzes einer Person nach einem der vorangehenden Ansprüche, **dadurch gekennzeichnet, dass** der Sturz nach dem Überschreiten einer Schwelle (S) durch eine numerische und gewichtete Kombination der Kriterien gemeldet wird.

10. Verfahren zur Erfassung des Sturzes einer Person nach einem der vorangehenden Ansprüche, **dadurch gekennzeichnet, dass** es eine Konversion von Messungen gemäß einer Ausrichtung des Sensors (2) umfasst, um die Messungen in einem an die Person gebundenen Achsenbezugssystem mit einer Vertikalachse, einer Anteriorposterior-Achse und einer Mediolateralachse auszudrücken, und eine Kalibrierung des Sensors zur Bestimmung der genannten Ausrichtung umfasst.
